(19) Europäisches Patentamt / European Patent Office / Office européen des brevets

(11) **EP 3 140 039 B1**

(12) **EUROPÄISCHE PATENTSCHRIFT**

(45) Veröffentlichungstag und Bekanntmachung des Hinweises auf die Patenterteilung:
**12.08.2020   Patentblatt 2020/33**

(21) Anmeldenummer: **15720724.2**

(22) Anmeldetag: **06.05.2015**

(51) Int Cl.:
**B01J 37/04** (2006.01)     **B01J 23/00** (2006.01)
**B01J 23/887** (2006.01)    **B01J 23/888** (2006.01)
**B01J 37/00** (2006.01)     **C07C 5/333** (2006.01)
**B01J 23/83** (2006.01)     **C07C 15/46** (2006.01)

(86) Internationale Anmeldenummer:
**PCT/EP2015/059908**

(87) Internationale Veröffentlichungsnummer:
**WO 2015/169826 (12.11.2015 Gazette 2015/45)**

(54) **VERBESSERTER KATALYSATOR FÜR DIE DEHYDRIERUNG VON KOHLENWASSERSTOFFEN**

IMPROVED CATALYST FOR DEHYDROGENATING HYDROCARBONS

CATALYSEUR DE DÉSHYDROGÉNATION D'HYDROCARBURES AMÉLIORÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.05.2014  EP 14167662**

(43) Veröffentlichungstag der Anmeldung:
**15.03.2017   Patentblatt 2017/11**

(73) Patentinhaber: **BASF SE**
**67056 Ludwigshafen am Rhein (DE)**

(72) Erfinder:
• **PATCAS, Florina Corina**
  **67065 Ludwigshafen (DE)**
• **DIETERLE, Martin**
  **67059 Ludwigshafen (DE)**

(74) Vertreter: **BASF IP Association**
**BASF SE**
**G-FLP-C006**
**67056 Ludwigshafen (DE)**

(56) Entgegenhaltungen:
**EP-A1- 2 106 852**

**Beschreibung**

Die vorliegende Erfindung betrifft einen Katalysator zur Dehydrierung von Kohlenwasserstoffen auf Basis von Eisenoxid sowie ein Verfahren zu dessen Herstellung. Der Katalysator enthält mindestens eine Eisenverbindung, mindestens eine Kaliumverbindung und 11 bis 24 Gew.-% mindestens einer Cerverbindung, gerechnet als $CeO_2$, wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist, und z eine Zahl von 2 bis 34 ist, und wobei der Katalysator 65 bis 89 Gew.-%, bezogen auf den gesamten Katalysator, der K/Fe-Mischoxidphasen enthält, wobei der Katalysator Cerdioxid-Kristallite mit einem mittleren Durchmesser von 10 bis 30 nm enthält, und wobei mindestens 50 Gew.-% des Cers, bezogen auf die gesamte Menge des eingesetzten Cers, gerechnet als $CeO_2$, in Form dieser Cerdioxid-Kristalliten vorliegen.

Weiterhin betrifft die vorliegende Erfindung ein Verfahren zur katalytischen Dehydrierung von Kohlenwasserstoffen unter Verwendung des erfindungsgemäßen Katalysators.

Im Stand der Technik ist seit langem der Einsatz von Eisenoxid-basierten Katalysatoren bei der Dehydrierung verschiedener Kohlenwasserstoffe zu den entsprechenden ungesättigten Kohlenwasserstoffen bekannt. Großtechnisch von Bedeutung sind beispielsweise die Dehydrierung von Ethylbenzol zu Styrol, von Isopropylbenzol zu alpha-Methylstyrol, von Buten zu Butadien oder von Isoamylen zu Isopren. Die Herstellung von Styrol durch heterogen katalysierte Dehydrierung von Ethylbenzol in Gegenwart von Wasserdampf ist ein großtechnisch bereits seit Beginn der dreißiger Jahre durchgeführtes Verfahren und hat sich als Syntheseweg für Styrol durchgesetzt. Styrol stellt eines der wichtigsten Monomere der Kunststoffindustrie dar und wird beispielsweise für die Herstellung von Polystyrol, Acrylnitril-Butadien-Styrol-Polymer (ABS) und synthetischem Kautschuk verwendet.

Die im Stand der Technik beschriebenen Eisenoxid-basierten Dehydrier-Katalysatoren sind in der Regel Multikomponenten-Systeme und enthalten im wesentlichen Eisenoxid und eine Alkalimetallverbindung, welche bei der Katalysatorherstellung beispielsweise als Alkalimetalloxid, -carbonat oder -hydroxid eingesetzt wird. Darüber hinaus enthalten diese Katalysatoren in der Regel verschiedene weitere aktive Komponenten (Promotoren), beispielsweise Oxide der Elemente der 5. und 6. Nebengruppe des Periodensystems oder der Seltenen Erden. Beispielsweise ist der Einsatz von Cerverbindungen als Promotor für solche Eisenoxid-basierten Dehydrier-Katalysatoren im Stand der Technik beschrieben.

Die katalytische Dehydrierung von aliphatischen oder alkylaromatischen Kohlenwasserstoffen wird großtechnisch üblicherweise in Gegenwart von Wasserdampf bei Temperaturen im Bereich von 500 bis 700 °C durchgeführt. Typischerweise werden in diesen Verfahren der Kohlenwasserstoff und der Wasserdampf gemischt und bei höheren Temperaturen über den Eisenoxid-Dehydrier-Katalysator geleitet.

Als eine Folge von Koksbildung werden im Laufe des Dehydrier-Verfahrens typischerweise die aktiven Zentren des Dehydrier-Katalysators blockiert, und es kommt zu einer allmählichen Deaktivierung des Katalysators. Um diese Deaktivierung zu vermindern wird dem Kohlenwasserstoff in der Regel Wasserdampf zugesetzt. Durch den Wasserdampf kann der auf der Katalysatoroberfläche entstandenen Koks in-situ vergast werden, wodurch die aktive Katalysatoroberfläche regeneriert werden kann. Daneben hat der Wasserdampf typischerweise noch die folgenden Funktionen: Lieferung der benötigten Reaktionswärme für die endotherme Dehydrierungsreaktion, Verschiebung des Gleichgewichtes zur Produktseite durch Verringerung der partiellen Drücke der Edukte, Aufrechterhaltung des Oxidationszustandes des Eisens gegen die reduzierende Wirkung von Wasserstoff und Kohlenwasserstoff.

Im Stand der Technik sind zahlreiche Dehydrier-Katalysatoren auf der Basis von Eisenoxid beschrieben.

EP-A 0 181 999 beschreibt Dehydrier-Katalysatoren enthaltend Eisenoxid, Kaliumoxid, Magnesiumoxid und optional weitere Metallverbindungen. Es wird unter anderem die optionale Zugabe von 0 bis 10 Gew.-% einer Verbindung des Cers, Molybdäns, Wolframs oder deren Gemische beschrieben. Die Beispiele von EP-A 0 181 999 beschreiben Dehydrier-Katalysator, welche etwa 6 Gew.-% Cerdioxid enthalten. Die in EP-A 0 181 999 beschriebenen Katalysatoren soll sich insbesondere durch eine verbesserte Stabilität gegen Siedewasser auszeichnen.

Die wissenschaftliche Publikation Hirano (Appl. Catal. 28, 1986, p. 119-130) untersucht den Einsatz von Cer als Promotor in einem Eisenoxid-Kaliumoxid-Katalysator. Die Publikation beschreibt, dass durch den Zusatz von 5 Gew.-% Cerdioxid in einem Eisenoxid-Kaliumoxid-Katalysator die Aktivierungsenergie bei der Umsetzung von Ethylbenzol zu Styrol abnimmt und die Reaktionsgeschwindigkeit zunimmt.

Das Dokument US 4,460,706 beschreibt Dehydrier-Katalysatoren enthaltend 40 bis 87,5 Gew.-% $Fe_2O_3$, 11 bis 50 Gew.-% Ceroxid, gerechnet als $Ce_2O_3$ und 1,5 bis 40 Gew.-% $K_2O$. In den experimentellen Beispielen von US 4,460,706 werden Ergebnisse der katalytischen Dehydrierung unter Verwendung von Katalysatoren mit unterschiedlichem Anteil an $Ce_2O_3$ beschreiben, wonach sich ein hinsichtlich der Styrol-Ausbeute optimaler Ceroxid-Gehalt, gerechnet als $Ce_2O_3$, von 13,2 Gew.-%, entsprechend etwa 13,8 Gew.-% $CeO_2$, ergibt. Die Anwesenheit von Kalium-Eisen-Mischoxidphasen in den Dehydrier-Katalysatoren wird nicht beschrieben.

Das Dokument US 4,758,543 beschreibt Dehydrier-Katalysatoren enthaltend 5 bis 30 Gew.-% Eisen, als $Fe_2O_3$, 40 bis 60 Gew.-% Kalium, als $K_2CO_3$, und 10 bis 60 Gew.-% Cer, als $Ce_2O_3$. In den Beispielen von US 4,758,543 werden bevorzugte Katalysatoren mit etwa 10 bis 20 Gew.-% Cer, als $Ce_2O_3$, beschrieben. Die Anwesenheit von Kalium-Eisen-Mischoxidphasen in den Dehydrier-Katalysatoren wird nicht beschrieben.

Das Dokument EP 0 894 528 beschreibt Katalysatoren zur Dehydrierung von Ethylbenzol enthaltend Eisenoxide, Kaliumoxid, Magnesium- und/oder Calciumoxid, Ceroxid sowie Wolfram und/oder Molybdänoxid, wobei der Katalysator Kaliumferrate mit einer Kristallitgröße kleiner 2 $\mu$m enthalten soll. Das Ceroxid, gerechnet als $CeO_2$, kann im Bereich von 2 bis 10 Gew.-% enthalten sein. Die Beispiele von EP 0 894 528 beschreiben Katalysatoren, die 7 Gew.-% $CeO_2$ enthalten. Die Katalysatoren werden gemäß EP 0 894 528 in einem 2-stufigen Verfahren hergestellt, um die Bildung der beschriebenen Kaliumferrate zu ermöglichen.

Das Dokument US 6,551,958 offenbart Katalysatoren mit 50 bis 90 Gew.-% $Fe_2O_3$, 1 bis 40 Gew.-% $K_2O$, 5 bis 20 Gew.-% $Ce_2O_3$, 0,1 bis 10 Gew.-% MgO und 1 bis 10 Gew.-% CaO. Der Katalysator gemäß US 6,551,958 kann ein oder mehreren Mischoxide der Summenformel $K_2O \cdot (Fe_2O_3)_n$, mit n ist eine natürliche Zahl zwischen 1 und 11, beinhaltet.

Das Dokument EP 2 106 852 A1 (Beispiel 1) offenbart einen Dehydrierkatalysator enthaltend 66,0 Gew.-% $Fe_2O_3$, 9,5 Gew.-% $K_2O$; 20,0 Gew.-% CeO; 2,0 Gew.-% CaO und 2.5 Gew.-% $MoO_3$. Der Katalysator wird hergestellt, indem Eisenoxid , Kaliumcarbonat, Calciumhydroxid, Molybdenoxide und Cercarbonathydroxid vermischt, geknetet, extrudiert und anschließend bei 900 °C kalziniert werden.

Es besteht ein Bedarf an weiter verbesserten Dehydrier-Katalysatoren für die Dehydrierung von Kohlenwasserstoffen, insbesondere für die Dehydrierung von Ethylbenzol, die eine verbesserte Katalysatoraktivität bei verbesserter oder gleichbleibender Stabilität und Standzeit aufweisen. Eine Aufgabe der vorliegenden Erfindung besteht darin, einen verbesserten Dehydrier-Katalysator auf der Basis von Eisenoxid bereitzustellen, der sich insbesondere durch eine verbesserte Katalysatoraktivität, insbesondere durch eine erhöhte Ausbeute auszeichnet. Ebenso sollen eine ausreichende mechanische Stabilität des Katalysators und eine Beständigkeit gegen Siedewasser gegeben sein.

Eine weitere Aufgabe der vorliegenden Erfindung besteht darin, Verfahren zur Herstellung der verbesserten Dehydrier-Katalysatoren bereitzustellen, welche einfach, kostengünstig und verlässlich durchzuführen sind, insbesondere sollten möglichst wenige und keine aufwändigen Verfahrensschritte, wie beispielsweise ein Sol-Gel-Verfahren, erforderlich sein.

Es wurde nun überraschenderweise gefunden, dass eine weitere Verbesserung der katalytischen Eigenschaften durch eine Optimierung des Cergehaltes in Kombination mit einem hohen Anteil an K/Fe-Mischoxidphasen erreicht werden kann. Es wurde gefunden, dass optimierte Dehydrier-Ausbeuten, insbesondere Styrol-Ausbeuten, erhalten werden können, wenn die Katalysatoren einen hohen Gehalt an K/Fe-Mischoxidphasen von 65 bis 89 Gew.-% aufweisen und gleichzeitig 11 bis 24 Gew.-% mindestens einer Cerverbindung, gerechnet als $CeO_2$, enthalten. Es konnte gezeigt werden, dass eine Kombination beider Merkmale erforderlich ist, um hohe Katalysatoraktivitäten, bei gleichzeitig guter mechanischer und chemischer Stabilität zu gewährleisten. Es konnte gezeigt werden, dass - entgegen des bisherigen Standes der Technik - der Gehalt an Cerdioxid als Aktivitätspromotor nicht über ein bestimmtes Ausmaß gesteigert werden sollte, bei dem der Gehalt der K/Fe-Mischoxidphasen unter dem bevorzugten Bereich absinkt.

Gegenstand der Erfindung ist ein Dehydrier-Katalysator enthaltend mindestens eine Eisenverbindung, mindestens eine Kaliumverbindung und 11 bis 24 Gew.-%, bevorzugt 12,5 bis 22 Gew.-%, besonders bevorzugt 15 bis 20 Gew.-%, bezogen auf den gesamten Katalysator, mindestens einer Cerverbindung, gerechnet als $CeO_2$, wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist und z eine Zahl von 2 bis 34 ist, wobei der Katalysator 65 bis 89 Gew.-%, bezogen auf den gesamten Katalysator, der einen oder mehreren K/Fe-Mischoxidphasen enthält, wobei der Katalysator Cerdioxid-Kristallite mit einem mittleren Durchmesser von 10 bis 30 nm, enthält, wobei mindestens 50 Gew.-% des Cers, bezogen auf die gesamte Menge des eingesetzten Cers, gerechnet als $CeO_2$, in Form von Cerdioxid-Kristalliten mit einem mittleren Durchmesser von 10 bis 30 nm vorliegen, und wobei der mittlere Durchmesser mittels Röntgen-Diffraktometrie bestimmt wird.

Soweit nicht anders angegeben, beziehen sich alle folgenden Mengenangaben auf Gew.-% und auf den gesamten Dehydrier-Katalysator und sind jeweils gerechnet auf das Metalloxid in der höchsten Oxidationsstufe. Im Sinne der vorliegenden Erfindung ist ppm als Milligramm pro Kilogramm (mg/kg) zu verstehen.

Die erfindungsgemäßen Katalysatoren zeichnen sich durch eine verbesserte Aktivität aus im Vergleich zu den im Stand der Technik beschriebenen Katalysatoren.

Dehydrier-Katalysator enthaltend mindestens eine Eisenverbindung, mindestens eine Kaliumverbindung, mindestens eine Cerverbindung und optional weitere Metallverbindungen ist im Sinne der vorliegenden Erfindung so zu verstehen, dass die entsprechenden Metalle in den gegebenenfalls angegebenen Mengen im Katalysator bestimmt werden können. Im Katalysator können typischerweise Mischphasen (z.B. Oxid-Mischphasen) und/oder isolierte Phasen der im Folgenden beschriebenen Metallverbindungen vorliegen. Es ist auch möglich das eine oder mehrere der im folgenden beschriebenen Komponente(n) teilweise oder vollständig in einem anderen bei der Katalysator-Herstellung verwendeten Rohstoff enthalten ist/sind.

Erfindungsgemäß enthält der Dehydrier-Katalysator mindestens eine Eisenverbindung bzw. wird bei der Herstellung des Dehydrier-Katalysators mindestens eine Eisenverbindung eingesetzt. Bevorzugt ist die mindestens eine Eisenverbindung ausgewählt aus natürlichen oder synthetischen Eisenoxiden und/oder Eisenoxidhydroxiden. Insbesondere ist die mindestens eine Eisenverbindung ausgewählt aus der Gruppe bestehend aus $\alpha$-$Fe_2O_3$ (Hämatit), $\gamma$-$Fe_2O_3$, Eisenoxidhydroxid (z.B. $\alpha$-FeOOH, Goethit) und $Fe_3O_4$ (Magnetit). Als synthetische Eisenoxide können beispielsweise Eisenoxide eingesetzt werden, die durch thermische Zersetzung von Eisensalzlösungen hergestellt wurden.

Bevorzugt wird als Eisenverbindung ein Eisenoxid, insbesondere $Fe_2O_3$, bevorzugt $\alpha$-$Fe_2O_3$ (Hämatit) eingesetzt. Bevorzugt ist auch der Einsatz von $\alpha$-$Fe_2O_3$ (Hämatit) in Kombination mit Goethit (FeOOH) und/oder Magnetit ($Fe_3O_4$) als Eisenverbindung. Der Anteil an Goethit (FeOOH) und/oder Magnetit ($Fe_3O_4$) beträgt dann typischerweise 0 bis 30 Gew.-%, bezogen auf die Gesamtmenge der Eisenverbindungen.

Die spezifische Oberfläche der Eisenverbindung kann beispielsweise mittels BET-Methode bestimmt werden und liegt typischerweise im Bereich von 1 bis 50 $m^2$/g, bevorzugt von 1 bis 20 $m^2$/g.

Die mindestens eine Eisenverbindung ist typischerweise in einer Menge im Bereich von 50 bis 80 Gew.-%, bevorzugt 60 bis 80 Gew.-%, besonders bevorzugt von 65 bis 75 Gew.-%, gerechnet als $Fe_2O_3$, im Dehydrier-Katalysator enthalten (bezogen auf das Gesamtgewicht des Dehydrier-Katalysators).

Erfindungsgemäß enthält der Katalysator mindestens eine Kaliumverbindung bzw. wird bei der Herstellung des Dehydrier-Katalysators mindestens eine Kaliumverbindung eingesetzt. Die mindestens eine Kaliumverbindung ist bevorzugt ausgewählt aus Kaliumoxid, Kaliumcarbonat, Kaliumhydroxid, Kaliumhydrogencarbonat, Kaliumoxalat und K/Fe-Mischoxidphasen, insbesondere ausgewählt aus Kaliumoxid, Kaliumcarbonat, Kaliumhydroxid und Kaliumhydrogencarbonat. Die mindestens eine Kaliumverbindung ist insbesondere ein Kaliumoxid ($K_2O$) oder ein Mischoxid. Es kann auch eine andere in der Wärme zersetzbaren Kaliumverbindung verwendet werden. Die mindestens eine Kaliumverbindung kann in dem Katalysator typischerweise als Oxid-Mischphase mit den im Katalysator vorhandenen Metallen vorliegen.

Die mindestens eine Kaliumverbindung ist typischerweise in einer Menge im Bereich von 1 bis 30 Gew.-%, bevorzugt von 5 bis 25 Gew.-%, insbesondere bevorzugt von 10 bis 15 Gew.-%, bezogen auf das Gesamtgewicht des Dehydrier-Katalysators und gerechnet als $K_2O$, im Dehydrier-Katalysator enthalten.

Das Kalium liegt bevorzugt in Form von einer oder mehreren unten beschriebenen K/Fe-Mischoxidphasen, in Form von Mischoxiden mit anderen Katalysatorkomponenten, insbesondere Promotoren, beispielsweise in Form von Kaliummolybdat, Kaliumwolframat, Kaliumvanadat, Kaliummanganat, und/oder in Form von reinen Kalium-Phasen, beispielsweise Kaliumoxid oder Kaliumcarbonat, im fertigen Katalysator vor.

Erfindungsgemäß liegt die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vor, wobei x eine Zahl von 1 bis 17 ist; y eine Zahl von 1 bis 22 ist, und z eine Zahl von 2 bis 34 ist. Oftmals werden diese K/Fe-Mischoxide bzw. K/Fe-Mischoxidphasen als Kaliumferrite bzw. Kaliumferritphasen oder als Kaliumferrate bzw. Kaliumferratphasen bezeichnet.

Unter K/Fe-Mischoxidphasen versteht man im Sinne der vorliegenden Erfindung ein oder mehrere Kalium-Eisen-Mischoxide der Summenformel $K_2O \cdot n\ Fe_2O_3$, wobei n eine Zahl von 0,1 bis 11 ist. Die Kalium-Eisen-Mischoxide können auch in der Form $K_aFe_bO_c$ beschrieben werden, wobei a, b und c natürliche Zahlen sind mit a = 1-17, b = 1-22 und c = 2-34. Bei den K/Fe-Mischoxidphasen kann es sich insbesondere um mindestens eine Verbindungen ausgewählt aus der Gruppe bestehend aus $KFeO_2$, $K_2Fe_2O_4$ ($K_2O \cdot Fe_2O_3$) (mit n = 1); $K_2Fe_8O_{13}$ ($K_2O \cdot 4\ Fe_2O_3$) (mit n=4); $K_2Fe_{10}O_{16}$ ($K_2O \cdot 5\ Fe_2O_3$) (mit n = 5), $K_2Fe_{22}O_{34}$ ($K_2O \cdot 11\ Fe_2O_3$) (mit n = 11), $K_6Fe_2O_5$, $K_6Fe_2O_6$, $K_9(FeO_4)_2$ (Raumgruppe C2/c) und $K_{17}Fe_5O_{16}$ (Raumgruppe Cm) handeln. Bevorzugt handelt es sich bei den K/Fe-Mischoxidphasen um mindestens eine Verbindungen ausgewählt aus der Gruppe bestehend aus $K_2Fe_2O_4$ ($K_2O \cdot Fe_2O_3$) (mit n = 1); $K_2Fe_8O_{13}$ ($K_2O \cdot 4\ Fe_2O_3$) (mit n=4); $K_2Fe_{10}O_{16}$ ($K_2O \cdot 5\ Fe_2O_3$) (mit n = 5) und $K_2Fe_{22}O_{34}$ ($K_2O \cdot 11\ Fe_2O_3$) (mit n = 11).

Die oben beschriebenen K/Fe-Mischoxidphasen und Mischungen davon können durch die allgemeine Formel $K_xFe_yO_z$, beschrieben werden, wobei x eine Zahl zwischen 1 und 17 ist, y eine Zahl zwischen 1 und 22 ist und z eine Zahl zwischen 2 und 34 ist.

K/Fe-Mischoxidphasen können durch die Reaktion einer Eisenverbindung, z.B. Eisenoxid, Eisenhydroxid, Eisenoxihydroxid oder andere Eisensalzen, und einer Kaliumverbindung, z.B. Kaliumoxid, Kaliumhydroxid oder anderen Kaliumsalzen, bei höheren Temperaturen entstehen. Die kristalline Zusammensetzung des Katalysators kann röntgenographisch qualitativ und quantitativ bestimmt werden.

Erfindungsgemäß enthält der Dehydrier-Katalysator 65 bis 85 Gew.-%, bezogen auf den gesamten Katalysator, der einen oder mehreren K/Fe-Mischoxidphasen.

Bevorzugt sind Dehydrier-Katalysatoren, bei denen der überwiegende Teil des bei der Herstellung eingesetzten Eisens in Form von einer oder mehreren K/Fe-Mischoxidphasen der allgemeinen Formel $K_xFe_yO_z$, wie oben beschrieben, vorliegt. Es ist möglich, dass ein gewisser Anteil des Eisens in Form von anderen Eisenoxiden vorliegt, beispielsweise in Form von Eisenoxid-Phasen wie FeOOH, $Fe_2O_3$, insbesondere $\alpha$-$Fe_2O_3$ (Hämatit), und $Fe_3O_4$, (Magnetit). Bevorzugt kann ein Anteil des eingesetzten Eisens als Hämatit ($\alpha$-$Fe_2O_3$) im fertigen Dehydrier-Katalysator vorliegen.

Insbesondere liegen mindestens 50 Gew.-%, bevorzugt mindestens 70 Gew.-%, insbesondere bevorzugt mindestens 80 Gew.-% des Eisens, bezogen auf die gesamte Menge des eingesetzten Eisens, gerechnet als $Fe_2O_3$, in Form von einer oder mehreren der oben beschriebenen K/Fe-Mischoxidphasen der allgemeinen Formel $K_xFe_yO_z$ vor. Insbesondere können 0 bis 50 Gew.-%, bevorzugt 0 bis 30 Gew.-%, insbesondere bevorzugt 0 bis 20 Gew.-% des eingesetzten Eisens, gerechnet als $Fe_2O_3$, in Form einer Hämatit-Phase ($\alpha$(alpha)-$Fe_2O_3$) vorliegen.

Erfindungsgemäß enthält der Dehydrier-Katalysator mindestens eine Cerverbindung bzw. wird bei der Herstellung des Dehydrier-Katalysators mindestens eine Cerverbindung eingesetzt. Die mindestens eine Cerverbindung ist bevorzugt ausgewählt aus Ceroxiden, Cerhydroxiden, Cercarbonaten, wasserhaltigen Cercarbonat und Ceroxalaten. Bevorzugt können Mischungen der genannten Cerverbindungen eingesetzt werden. Bevorzugt ist die mindestens eine Cerverbindung ausgewählt aus Cer(IV)-oxid ($CeO_2$), Cer(III)-oxid ($Ce_2O_3$), Cer(III)-oxalat und Cer(III)-carbonat, bevorzugt aus Cer(IV)-oxid ($CeO_2$) und Cer(III)-carbonat. Typischerweise wird die mindestens eine Cerverbindung bei der Herstellung des Katalysators in Cerdioxid, insbesondere in kristallines Cerdioxid, umgewandelt.

Erfindungsgemäß enthält der Dehydrier-Katalysator 11 bis 24 Gew.-%, bevorzugt 12,5 bis 22 Gew.-%, besonders bevorzugt 15 bis 20 Gew.-%, mindestens einer Cerverbindung, gerechnet als $CeO_2$.

Typischerweise ist Cer überwiegend in Form von Cerdioxid, insbesondere als kristallines Cerdioxid, insbesondere als kristallines Cerdioxid in Form von Cerianit im Katalysator enthalten. Das Cer kann gegebenenfalls zumindest teilweise auch als Mischverbindungen, insbesondere als Mischoxid, mit anderen Katalysatorkomponenten im Katalysator vorliegen.

Insbesondere enthält der Katalysator Cerdioxid-Kristallite, welche im Wesentlichen aus kristallinem Cerdioxid, bevorzugt aus kristallinem Cerdioxid in Form von Cerianit, bestehen und welche als fein disperse Kristallit-Körner mit einem mittleren Durchmesser von 10 bis 30 nm, bevorzugt von 12 und 25 nm, besonders bevorzugt von 14 bis 22 nm, ganz besonders bevorzugt von 16 bis 19 nm vorliegen.

Insbesondere liegen mindestens 50 Gew.-%, bevorzugt mindestens 60 Gew.-%, bevorzugt mindestens 80 Gew.-%, insbesondere bevorzugt mindestens 90 Gew.-% des Cers, bezogen auf die gesamte Menge des eingesetzten Cers, gerechnet als $CeO_2$, in Form des oben beschriebenen kristallinen Cerdioxids mit der beschriebenen Kristallitgröße vor.

In einer bevorzugten Ausführungsform enthält der oben beschriebene erfindungsgemäße Katalysator 11 bis 24 Gew.-%, bevorzugt 12,5 bis 22 Gew.-%, besonders bevorzugt 15 bis 20 Gew.-%, der mindestens einen Cerverbindung, gerechnet als $CeO_2$ und 50 bis 90 Gew.-%, bevorzugt 60 bis 80 Gew.-%, insbesondere bevorzugt 65 bis 75 Gew.-%, bezogen auf den gesamten Katalysator, der K/Fe-Mischoxidphasen.

Bevorzugt enthält der Dehydrier-Katalysator als weitere Komponenten mindestens eine Erdalkalimetallverbindung, bzw. es wird bei der Herstellung des Dehydrier-Katalysators mindestens eine Erdalkalimetallverbindung eingesetzt. Insbesondere kann der Dehydrier-Katalysator als weitere Komponente 0,1 bis 20 Gew.-%, bevorzugt 0,1 bis 10 Gew.-%, insbesondere bevorzugt 1 bis 5 Gew.-% mindestens einer Erdalkalimetallverbindung, gerechnet als Oxid, enthalten.

In einer bevorzugten Ausführungsform enthält der Dehydrier-Katalysator mindestens eine Magnesiumverbindung als weitere Komponente. Bevorzugt enthält der Dehydrier-Katalysator als weitere Komponente 0,1 bis 10 Gew.-%, bevorzugt 1 bis 5 Gew.-% mindestens einer Magnesiumverbindung, gerechnet als MgO. Insbesondere ist die mindestens eine Magnesiumverbindung ausgewählt aus Magnesiumoxid, Magnesiumcarbonat und Magnesiumhydroxid. Bevorzugt handelt es sich bei der mindestens einen Magnesiumverbindung um Magnesiumoxid

(MgO) und/oder Magnesiumcarbonat ($MgCO_3$). Bevorzugt wird bei der Herstellung des Katalysators als weitere Komponente Magnesiumoxid (MgO) und/oder Magnesiumcarbonat ($MgCO_3$) eingesetzt.

In einer bevorzugten Ausführungsform enthält der Dehydrier-Katalysator mindestens eine Calciumverbindung als weitere Komponente. Bevorzugt enthält der Dehydrier-Katalysator als weitere Komponente 0,1 bis 10 Gew.-%, bevorzugt 1 bis 5 Gew.-% mindestens einer Calciumverbindung, gerechnet als CaO. Insbesondere ist die mindestens eine Calciumverbindung ausgewählt aus Calciumoxid, Calciumcarbonat und Calciumhydroxid. Bevorzugt handelt es sich bei der mindestens einen Calciumverbindung um Calciumoxid (CaO). Bevorzugt wird bei der Herstellung des Katalysators als weitere Komponente Calciumoxid (CaO) und/oder Calciumhydroxid ($Ca(OH)_2$) eingesetzt.

In einer bevorzugten Ausführungsform enthält der Dehydrier-Katalysator mindestens eine Magnesiumverbindung und mindestens eine Calciumverbindung. Insbesondere enthält der Dehydrier-Katalysator 0,1 bis 10 Gew.-%, bevorzugt 1 bis 5 Gew.-% mindestens einer Magnesiumverbindung, gerechnet als MgO, und 0,1 bis 10 Gew.-%, bevorzugt 1 bis 5 Gew.-% mindestens einer Calciumverbindung, gerechnet als CaO.

Eine bevorzugte Ausführungsform der vorliegenden Erfindung betrifft einen Dehydrier-Katalysator enthaltend

50 bis 80 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, besonders bevorzugt 10 bis 20 Gew.-%, mindestens einer Kaliumverbindung, gerechnet als $K_2O$;

11 bis 24 Gew.-%, bevorzugt 12,5 bis 22 Gew.-%, besonders bevorzugt 15 bis 20 Gew.-%, mindestens einer Cerverbindung, gerechnet als $CeO_2$;

0,1 bis 10 Gew.-%, bevorzugt 0,1 bis 5 Gew.-%, mindestens einer Magnesiumverbindung, gerechnet als MgO;

0,1 bis 10 Gew.-%, bevorzugt 0,1 bis 5 Gew.-%, mindestens einer Calciumverbindung, gerechnet als CaO;

0 bis 30 Gew.-%, bevorzugt 0,0001 bis 10 Gew.-%, mindestens einer weiteren Komponente.

In einer bevorzugten Ausführungsform addieren sich die oben genannten Komponenten zu 100 Gew.-%.

Die weiteren Komponenten können in Mengen von 0 bis 30 Gew.-%, bevorzugt von 0 bis 20 Gew.-%, bevorzugt von 0,0001 bis 10 Gew.-%, insbesondere bevorzugt von 0,001 bis 5 Gew.-%, insbesondere bevorzugt von 0,5 bis 5 Gew.-% enthalten sein bzw. bei der Herstellung des Katalysators zugesetzt werden.

Typischerweise kann der Dehydrier-Katalysator als mindestens eine weitere Komponente, insbesondere als Promotor oder Dotierstoff, eine oder mehrere der üblichen Verbindungen zur Steigerung der Aktivität und/oder Selektivität enthalten. Beispielsweise kann als weitere Komponente mindestens eine Verbindung ausgewählt aus Verbindungen umfassend ein Metall ausgewählt aus der Gruppe bestehend aus Mn, Ti, Cr, Co, Ni, Cu, Zn, Al, Ga, Ge, Zr, Nb, Ru, Rh, Pd, Ag, Cd, In, Sn, Sb, La, Hf, Ta, Re, Ir, Pt, Au, Pb und Bi enthalten sein, bzw. es wird bei der Herstellung des Dehydrier-Katalysators mindestens eine solche Verbindung eingesetzt. Es ist weiterhin möglich, dass eine oder mehrere der oben genannten Komponente(n) bereits teilweise oder vollständig in einem anderen Rohstoff enthalten ist/sind, der bei der Katalysatorherstellung verwendetet wird, z.B. im Eisenoxid. Die genannten üblichen Komponenten, insbesondere Promotoren oder Dotierstoffe, können typischerweise in Mengen von 0 bis 10 Gew.-%, vorzugsweise von 0,0001 bis 5 Gew.-%, vorzugsweise von 0,001 bis 2 Gew.-%, gerechnet jeweils als Oxid in der höchsten Oxidationsstufe, bezogen auf den gesamten Katalysator, enthalten sein.

Bevorzugt kann der Dehydrier-Katalysator als weitere Komponente mindestens eine Verbindung ausgewählt aus Verbindungen umfassend ein Metall ausgewählt aus der Gruppe bestehend aus Molybdän (Mo), Titan (Ti), Vanadium (V) und Wolfram (W), enthalten; bzw. es wird bei der Herstellung des Dehydrier-Katalysators mindestens eine solche Verbindung eingesetzt. Die weitere Komponente kann insbesondere ausgewählt werden aus Sauerstoffverbindungen (beispielsweise Oxiden, Oxidhydraten, Oxoverbindungen von Molybdän (Mo), Titan (Ti), Vanadium (V) und Wolfram (W). Insbesondere handelt es sich bei der mindestens eine Verbindung ausgewählt aus Verbindungen umfassend ein Metall ausgewählt aus der Gruppe bestehend aus Molybdän (Mo), Titan (Ti), Vanadium (V) und Wolfram (W) um eine Verbindung, die unter Hitzeeinwirkung bei der Herstellung des Dehydrier-Katalysators zerfällt.

Bevorzugt enthält der Dehydrier-Katalysator als weitere Komponente 0,0001 bis 10 Gew.-%, bevorzugt 0,001 bis 5 Gew.-%, besonders bevorzugt von 0,02 bis 5 Gew.-%, mindestens einer Verbindung ausgewählt aus der Gruppe bestehend aus Molybdän (Mo), Titan (Ti), Vanadium (V) und Wolfram (W), gerechnet als Oxid in der jeweils höchsten Oxidationsstufe.

Bevorzugt enthält der Dehydrier-Katalysator als weitere Komponente 0,0001 bis 10 Gew.-%, bevorzugt 0,001 bis 5 Gew.-%, besonders bevorzugt von 0,02 bis 5 Gew.-%, mindestens einer Verbindung ausgewählt aus Verbindungen umfassend ein Metall ausgewählt aus der Gruppe bestehend aus Molybdän (Mo), Titan (Ti), Vanadium (V) und Wolfram (W), gerechnet als Oxid in der jeweils höchsten Oxidationsstufe.

Bevorzugt wird als mindestens eine weitere Komponente mindestens eine Molybdänverbindung eingesetzt, ausgewählt aus Molybdänoxiden und Molybdaten (z.B. Ammoniummolybdat, Kaliummolybdat). Bevorzugt handelt es sich bei der mindestens einen Molybdänverbindung um Molybdänoxid.

Insbesondere bevorzugt enthält Dehydrier-Katalysator als weitere Komponente 0,1 bis 10 Gew.-%, bevorzugt 0,5 bis 5 Gew.-%, insbesondere bevorzugt 1 bis 4 Gew.-%, mindestens einer Molybdänverbindung, gerechnet als $MoO_3$.

Insbesondere bevorzugt enthält der Dehydrier-Katalysator als weitere Komponente mindestens eine Titanverbindung; bzw. es kann bei der Herstellung des Dehydrier-Katalysators mindestens eine Titanverbindung eingesetzt werden. Der Dehydrier-Katalysator kann 0 bis 1.000 ppm, insbesondere 1 bis 1.000 ppm, bevorzugt 10 bis 500 ppm, bevorzugt 30 bis 500 ppm, besonders bevorzugt 50 bis 220 ppm, der mindestens einen Titanverbindung, gerechnet als $TiO_2$, bezogen auf den gesamten Katalysator, enthalten.

Die mindestens eine Titanverbindung kann insbesondere ausgewählt sein aus Titanoxiden, Titanalkoholaten und Titancarboxylaten. Bevorzugt ist die mindestens eine Titanverbindung Titandioxid ($TiO_2$). Die mindestens eine Titanverbindung wird bei der Herstellung des Katalysators bevorzugt als Titandioxid ($TiO_2$) zugegeben. Es ist aber auch möglich andere Titanverbindungen einzusetzen. Es ist weiterhin möglich, dass die mindestens eine Titanverbindung bereits teilweise oder vollständig in einem anderen bei der Katalysatorherstellung verwendeten Rohstoff enthalten ist, z.B. im Eisenoxid.

Insbesondere kann der Dehydrier-Katalysator als weitere Komponente 0,1 bis 10 Gew.-%, besonders bevorzugt 1 bis 5 Gew.-%, mindestens einer Vanadiumverbindung, gerechnet als $V_2O_5$, enthalten.

In einer bevorzugten Ausführungsform bezieht sich die vorliegende Erfindung auf einen Dehydrier-Katalysator wie oben beschrieben enthaltend:

50 bis 80 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, besonders bevorzugt 10 bis 20 Gew.-%, mindestens einer Kaliumverbindung, gerechnet als $K_2O$;

11 bis 24 Gew.-%, bevorzugt 12,5 bis 22 Gew.-%, besonders bevorzugt 15 bis 20 Gew.-%, mindestens einer Cerverbindung, gerechnet als $CeO_2$;

0,1 bis 10 Gew.-%, bevorzugt 1 bis 5 Gew.-%, mindestens einer Magnesiumverbindung gerechnet als $MgO$;

0,1 bis 10 Gew.-%, bevorzugt 1 bis 5 Gew.-%, mindestens einer Calciumverbindung, gerechnet als $CaO$;

0,0001 bis 10 Gew.-%, bevorzugt 0,001 bis 5 Gew.-%, mindestens einer Verbindung ausgewählt aus Verbindungen umfassend ein Metall ausgewählt aus der Gruppe bestehend aus Molybdän (Mo), Titan (Ti), Vanadium (V) und Wolfram (W), jeweils gerechnet als das Oxid in der höchsten Oxidationsstufe.

In einer bevorzugten Ausführungsform addieren sich die oben genannten Komponenten zu 100 Gew.-%.
In einer bevorzugten Ausführungsform bezieht sich die vorliegende Erfindung auf einen Dehydrier-Katalysator wie oben beschrieben enthaltend:

50 bis 80 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, besonders bevorzugt 10 bis 20 Gew.-%, mindestens einer Kaliumverbindung, gerechnet als $K_2O$;

11 bis 24 Gew.-%, bevorzugt 12,5 bis 22 Gew.-%, besonders bevorzugt 15 bis 20 Gew.-%, mindestens einer Cerverbindung, gerechnet als $CeO_2$;

0,1 bis 10 Gew.-%, bevorzugt 1 bis 5 Gew.-% mindestens einer Magnesiumverbindung gerechnet als $MgO$;

0,1 bis 10 Gew.-%, bevorzugt 1 bis 5 Gew.-%, mindestens einer Calciumverbindung, gerechnet als $CaO$;

0,1 bis 10 Gew.-%, bevorzugt 0,1 bis 5 Gew.-%, insbesondere bevorzugt 1 bis 4 Gew.-%, mindestens einer Molybdänverbindung, gerechnet als $MoO_3$;

1 bis 1.000 ppm, bevorzugt 10 bis 500 ppm, bevorzugt 30 bis 500 ppm, insbesondere bevorzugt 50 bis 220 ppm, mindestens einer Titanverbindung, gerechnet als $TiO_2$.

In einer bevorzugten Ausführungsform addieren sich die oben genannten Komponenten zu 100 Gew.-%.
Alle Angaben in Gew.-% beziehen sich - soweit nicht anders angegeben - auf den gesamten Dehydrier-Katalysator. Alle Angaben in Gew.-% wurden - soweit nicht anders angegeben - auf das Oxid des betreffenden Metalls jeweils in der höchsten Oxidationsstufe berechnet.
In einer Ausführungsform enthält der oben beschriebene Dehydrier-Katalysator als weitere Komponente mindestens eine weitere Seltenerdenmetallverbindung neben Cer, insbesondere ausgewählt aus der Gruppe bestehend aus Lanthan (La), Praseodym (Pr) und Neodym (Nd). Bevorzugt enthält der Dehydrier-Katalysator 1 bis 1.000 ppm, bevorzugt 10 bis 500 ppm, besonders bevorzugt 20 bis 300 ppm, mindestens einer weiteren Seltenerdenmetallverbindung, neben Cer, gerechnet als Oxid in der jeweils höchsten Oxidationsstufe. Insbesondere enthält der Katalysator 1 bis 1.000 ppm, bevorzugt 10 bis 500 ppm, besonders bevorzugt 20 bis 300 ppm, mindestens einer Seltenerdenmetallverbindung ausgewählt aus der Gruppe bestehend aus Lanthan, Praseodym und Neodym. Bevorzugt kann der Dehydrier-Katalysator als weitere Komponente 1 bis 1.000 ppm, bevorzugt 3 bis 500, besonders bevorzugt 10 bis 100 ppm mindestens einer Lanthanverbindung, gerechnet als $La_2O_3$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Praseodymverbindung, gerechnet als $PrO_2$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Neodymverbindung, gerechnet als $Nd_2O_3$, enthalten.

Der oben beschriebene Dehydrier-Katalysator kann vorzugsweise als weitere Komponente mindestens eine Verbindung von Metallen der 8. bis 12. Nebengruppe des Periodensystems enthalten. Bevorzugt enthält der oben beschriebene Dehydrier-Katalysator als weitere Komponente mindestens eine Verbindung von Metallen ausgewählt aus der Gruppe bestehend aus Mangan (Mn), Ruthenium (Ru), Osmium (Os), Kobalt (Co), Nickel (Ni), Palladium (Pd), Platin (Pt), Kupfer (Cu), Silber (Ag), Gold (Au) und Zink (Zn); bevorzugt ausgewählt aus der Gruppe bestehend aus Mangan (Mn), Kobalt (Co), Palladium (Pd), Kupfer (Cu), und Zink (Zn); besonders bevorzugt ausgewählt aus der Gruppe bestehend aus Mangan (Mn), Kupfer (Cu), und Zink (Zn). Der oben beschriebene Dehydrier-Katalysator kann als weitere Komponente insbesondere 1 bis 1.000 ppm, bevorzugt 50 bis 500 ppm, besonders bevorzugt 50 bis 200 ppm, mindestens einer Verbindung von Metallen der 8. bis 12. Nebengruppe des Periodensystems, gerechnet jeweils als Oxid in der höchsten Oxidationsstufe, enthalten. In einer bevorzugten Ausführungsform enthält der oben beschriebene Dehydrier-Katalysator 1 bis 1.000 ppm, bevorzugt 50 bis 500 ppm, besonders bevorzugt 50 bis 200 ppm, mindestens einer Verbindung von Metallen ausgewählt aus der Gruppe bestehend aus Mangan (Mn), Kupfer (Cu), und Zink (Zn), gerechnet jeweils als das Oxid in der höchsten Oxidationsstufe. Bevorzugt kann der Dehydrier-Katalysator als weitere Komponente 1 bis 2.000 ppm, bevorzugt 30 bis 2.000 ppm, besonders bevorzugt 100 bis 2.000 ppm, mindestens einer Manganverbindung, gerechnet als $MnO_2$, enthalten. Bevorzugt kann der Dehydrier-Katalysator als weitere Komponente 1 bis 1.000 ppm, bevorzugt 10 bis 200, besonders bevorzugt 30 bis 100 ppm mindestens einer Kupferverbindung, gerechnet als CuO, enthalten. Bevorzugt kann der Dehydrier-Katalysator als weitere Komponente 1 bis 1.000 ppm, bevorzugt 1 bis 500 ppm, besonders bevorzugt 10 bis 100 ppm, mindestens einer Zinkverbindung, gerechnet als ZnO, enthalten.

Darüber hinaus kann der Dehydrier-Katalysator als weitere Komponente mindestens eine Verbindung von Elementen der 4. Hauptgruppe des Periodensystems enthalten. Bevorzugt enthält der oben beschriebene Dehydrier-Katalysator als weitere Komponente mindestens eine Verbindung ausgewählt aus der Gruppe bestehend aus Silicium (Si)-, Germanium (Ge)-, Zinn (Sn)-, und Blei (Pb)-Verbindungen, bevorzugt mindestens eine Siliciumverbindung. Insbesondere enthält der Dehydrier-Katalysator 1 bis 1.000 ppm, bevorzugt 5 bis 500 ppm, besonders bevorzugt 10 bis 100 ppm, mindestens einer Verbindung von ausgewählt aus der Gruppe bestehend aus Silicium (Si)-, Germanium (Ge)-, Zinn (Sn)-, und Blei (Pb)-Verbindungen, gerechnet als Oxid in der jeweils höchsten Oxidationsstufe. In einer Ausführungsform enthält der beschriebene Dehydrier-Katalysator 1 bis 1000 ppm, bevorzugt 5 bis 500 ppm, besonders bevorzugt 10 bis 100 ppm, mindestens einer Siliciumverbindung, gerechnet als $SiO_2$.

Typischerweise kann der oben beschriebene Dehydrier-Katalysator als Nichtmetall, neben Sauerstoff, mindestens ein Nicht-Metall ausgewählt aus Nichtmetallen der 5. bis 7. Hauptgruppe des Periodensystems enthalten, insbesondere ausgewählt aus der Gruppe bestehend aus Stickstoff, Phosphor, Schwefel und Chlor.

In einer weiteren Ausführungsform enthält der Dehydrier-Katalysator:

50 bis 80 Gew.-%, bevorzugt 60 bis 80 Gew.-%, mindestens einer Eisenverbindung, gerechnet als $Fe_2O_3$;

1 bis 30 Gew.-%, bevorzugt 5 bis 25 Gew.-%, besonders bevorzugt 10 bis 20 Gew.-%, mindestens einer Kaliumverbindung, gerechnet als $K_2O$;

11 bis 24 Gew.-%, bevorzugt 12,5 bis 22 Gew.-%, besonders bevorzugt 15 bis 20 Gew.-%, mindestens einer Cerverbindung, gerechnet als $CeO_2$;

0,1 bis 10 Gew.-%, bevorzugt 1 bis 5 Gew.-% mindestens einer Magnesiumverbindung gerechnet als MgO;

0,1 bis 10 Gew.-%, bevorzugt 1 bis 5 Gew.-%, mindestens einer Calciumverbindung, gerechnet als CaO;

0,1 bis 10 Gew.-%, bevorzugt 0,1 bis 5 Gew.-%, insbesondere bevorzugt 1 bis 4 Gew.-%, mindestens einer Molybdänverbindung, gerechnet als $MoO_3$;

1 bis 1.000 ppm, bevorzugt 10 bis 500 Gew.-%, bevorzugt 30 bis 500 ppm, insbesondere bevorzugt 50 bis 220 ppm, mindestens einer Titanverbindung, gerechnet als $TiO_2$;

0 bis 10 Gew.-%, besonders bevorzugt 1 bis 5 Gew.-%, mindestens einer Vanadiumverbindung, gerechnet als $V_2O_5$ und

1 bis 10.000 ppm, bevorzugt 10 bis 5.000 ppm, insbesondere bevorzugt 10 bis 3.000 ppm mindestens einer weiteren Komponente ausgewählt aus Lanthanverbindungen, Praseodymverbindungen, Neodymverbindungen, Nickelverbindungen, Kupferverbindungen, Zinkverbindungen und Siliciumverbindungen, gerechnet jeweils als Oxid in der höchsten Oxidationsstufe.

In einer bevorzugten Ausführungsform ergeben die oben genannten Komponenten 100 Gew.-%.

Alle Angaben in Gew.-% beziehen sich - soweit nicht anders angegeben - auf den gesamten Dehydrier-Katalysator. Alle Angaben in Gew.-% wurden - soweit nicht anders angegeben - auf das Oxid des betreffenden Metalls jeweils in der höchsten Oxidationsstufe berechnet.

Insbesondere bezieht sich die vorliegende Erfindung auf einen oben beschriebenen Dehydrier-Katalysator zur katalytischen Dehydrierung von Kohlenwasserstoffen bei einem molaren Dampf/Kohlenwasserstoff-Verhältnis 1 bis 20; bevorzugt im Bereich von 1 bis 10; insbesondere bevorzugt im Bereich von 1 bis 9, besonders bevorzugt von 5 bis 8,5.

Weiterhin bezieht sich die vorliegende Erfindung auf ein Verfahren zur Herstellung eines oben beschriebenen Dehydrier-Katalysators umfassend die folgenden Schritte:

i) Herstellen einer Katalysator-Vormischung durch Vermischen mindestens einer Eisenverbindung, mindestens einer Kaliumverbindung, 11 bis 24 Gew.-%, bevorzugt 12,5 bis 22 Gew.-%, besonders bevorzugt 15 bis 20 Gew.-%, bezogen auf den fertigen Katalysator, mindestens einer Cerverbindung, gerechnet als $CeO_2$; optional weiterer Metallverbindungen, optional weiterer Komponenten und optional mindestens eines Bindemittels mit einem Lösungsmittel;

ii) Herstellen von Katalysator-Formkörpern aus der in Schritt i) erhaltenen Katalysator-Vormischung;

iii) Trocknen der Katalysator-Formkörper und Kalzinieren der Katalysator-Formkörper.

Für das Verfahren zur Herstellung eines Dehydrier-Katalysators werden bevorzugt die oben im Zusammenhang mit dem Dehydrier-Katalysator beschriebenen Eisenverbindungen, Kaliumverbindungen, Cerverbindungen und weitere Komponenten, insbesondere weitere Metallverbindungen, bevorzugt in den beschriebenen Mengen, eingesetzt. Optional können die oben beschriebenen Komponenten bei der Herstellung eingesetzt werden, bevorzugt befindet sich eine oder mehrere der Komponenten ganz oder teilweise in einem der eingesetzten Rohstoffe, beispielsweise im verwendeten Eisenoxid und/oder Cercarbonat.

Die grundlegende Vorgehensweise bei der Herstellung von Dehydrier-Katalysatoren ist dem Fachmann bekannt. Die Herstellung der oben beschriebenen Dehydrier-Katalysatoren kann beispielsweise wie in WO 99/49966 oder US 6,551,958 beschrieben erfolgen.

Die Auswahl der Katalysator-Komponenten (Edukte, Rohstoffe) und die Wahl der Herstellbedingungen, insbesondere der Kalzinierungsbedingungen können so erfolgen, dass erfindungsgemäße Katalysatoren mit dem erfindungsgemäßen Anteil an K/Fe-Mischoxidphasen erhalten werden. Die beschriebenen K/Fe-Mischoxidphasen entstehen typischerweise durch die Reaktion von Eisen- und Kaliumverbindungen währen der Endkalzinierung des Katalysators in Anwesenheit aller anderer Komponenten des Katalysators. Es ist auch möglich in einem zweistufigen Verfahren zunächst ein Katalysator-Vorprodukt, das die Kalium-Ferritphasen enthält, aus Eisen- und Kaliumverbindungen ohne weitere Komponenten herzustellen und danach das Katalysator-Vorprodukt mit der Cerverbindung und den weiteren Komponenten zu vermischen und nochmals zu kalzinieren.

Besonders vorteilhaft ist ein Herstellverfahren, das möglichst wenige Schritte, insbesondere möglichst wenig Kalzinierungsschritte, aufweist.

Zur Herstellung der Katalysator-Vormischung werden in der Regel die Komponenten, typischerweise in Form von festen Pulvern, vermischt und dann mit einem Lösungsmittel, insbesondere Wasser, gegebenenfalls unter Zugabe eines Bindemittels vermischt. Das Vermischen erfolgt bevorzugt durch ein inniges Vermischen, z.B. durch Verkneten, in einem Rührkessel, Mixmuller, Mixer, Kneter oder Extruder, bevorzugt in einem Mixmuller, Kneter oder Mixer. Unter Lösungsmittel ist in diesem Zusammenhang insbesondere ein flüssiges Lösungs- und/oder Dispergiermittel zu verstehen, in dem die festen Katalysatorkomponenten dispergiert werden.

Als Lösungsmittel wird insbesondere Wasser oder eine Mischung aus Wasser und aus polaren Lösungsmitteln, z.B. Alkoholen, Estern, eingesetzt. Als Bindemittel (auch Plastifizierhilfsmittel) können beispielsweise Alginat, Stärke, Carboxymethylcellulose, Hydroxyethylcellulose und Polyvinylalkohol eingesetzt werden. Typischerweise werden die Bindemittel in Form einer Lösung in Wasser eingesetzt.

Aus der so erhaltenen Katalysator-Vormischung werden dann typischerweise Katalysator-Formkörper hergestellt, z.B. durch Extrusion oder Pressen, die anschließend getrocknet und kalziniert werden.

Die Herstellung von Katalysator-Formkörpern aus der Katalysator-Vormischung erfolgt typischerweise durch extrudieren oder pressen (Tablettenpressen). Beispiele für Katalysator-Formkörper sind Zylinder (Pellets), Ringe, Sternkörper und Wabenkörper. Bevorzugt erfolgt die Herstellung von Katalysator-Formkörpern aus der in Schritt i) erhaltenen Katalysator-Vormischung mittels Extrusion.

Nach der Formgebung werden die feuchten Formkörper typischerweise bei Temperaturen von 80 °C bis 500 °C, bevorzugt von 120 bis 350°C getrocknet. Die Trocknung kann beispielsweise im Trockenschank (z.B. auf Blechen), in einer Trocknungstrommel und/oder auf Bandtrockner stattfinden.

Anschließend werden die Formkörper typischerweise kalziniert. Die Kalzinierung kann in Muffelofen, Bandkalzinierer, Drehrohrofen usw. durchgeführt werden. Vorzugsweise werden die Katalysatorstränge in einem Drehrohrofen kalziniert.

Das Kalzinieren der Katalysator-Formkörper in Schritt iii) erfolgt bei einer Temperatur im Bereich von 750°C bis 850 °C.

Bevorzugt erfolgt das Kalzinieren der Katalysator-Formkörper in Schritt iii) bei einer Temperatur im Bereich von 750 °C bis 850 °C, und über einen Zeitraum von 10 min bis 300 min, bevorzugt von 30 min bis 300 min, besonders bevorzugt von 30 min bis 180 min.

Bevorzugt kann die Kalzinierung in Schritt iii) in einem stationären Verfahren, beispielsweise in einem Muffelofen, oder in einem kontinuierlichen Verfahren, beispielsweise in einem kontinuierlichen Drehrohrofen, erfolgen.

Als Verbindungen und weitere Komponenten können Verbindungen eingesetzt werden, wie sie im fertigen Katalysator vorliegen, oder Verbindungen, die sich während des Herstellungsprozess in Verbindungen umwandeln, wie sie im fertigen Katalysator vorliegen. Es werden bevorzugt die im Zusammenhang mit dem erfindungsgemäßen Dehydrier-Katalysator beschriebenen Verbindungen eingesetzt.

Insbesondere betrifft die Erfindung ein Verfahren zur Herstellung eines Dehydrier-Katalysators wie beschrieben, wobei eine Eisenverbindung eingesetzt wird, die zu mindestens 50 Gew.-%, bevorzugt zu mindestens 80 Gew.-%, bezogen auf die gesamte Eisenverbindung, Eisen(III)-oxid ($Fe_2O_3$) enthält. Als Eisenkomponente wird überwiegend Hämatit ($Fe_2O_3$) eingesetzt. Ein Teil des Eisens kann als Goethit (FeOOH) oder Magnetit ($Fe_3O_4$) eingesetzt werden, wobei dieser Anteil 0 bis 30% betragen kann. Insbesondere betrifft die Erfindung ein Verfahren zur Herstellung eines Dehydrier-Katalysators wie beschrieben, wobei als Kaliumverbindung ein Kaliumoxid, Kaliumhydroxid, Kaliumcarbonat und/oder Kaliumhydrogencarbonat eingesetzt werden. Bevorzugt werden als Cerverbindung Ceroxide, insbesondere Cerdioxid, Cercarbonate, Cerhydroxide, und/oder Cerhydrogencarbonate eingesetzt. Bevorzugt werden als Magnesiumverbindung Magnesiumoxid, Magnesiumcarbonat und/oder Magnesiumhydroxid eingesetzt. Bevorzugt werden als Calciumverbindung Calciumoxid, Calciumcarbonat und/oder Calciumhydroxid eingesetzt. Bevorzugt werden als Titanverbindung Titanoxide, insbesondere $TiO_2$, Titanalkoholate und/oder Titancarboxylate eingesetzt. Bevorzugt werden als Molybdänverbindung Molybdänoxide, insbesondere $MoO_3$ eingesetzt. Bevorzugt werden als Vanadiumverbindung Vanadiumoxide eingesetzt.

In einem weiteren Aspekt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem oben beschriebenen Dehydrier-Katalysator in Kontakt gebracht wird.

Die oben im Zusammenhang mit dem erfindungsgemäßen Dehydrier-Katalysator und mit dem Verfahren zu seiner Herstellung beschriebenen bevorzugten Ausführungsformen gelten in analoger Weise für das erfindungsgemäße Verfahren zur katalytischen Dehydrierung eines Kohlenwasserstoffs.

Das Verfahren zur katalytischen Dehydrierung eines Kohlenwasserstoffs unter Verwendung des erfindungsgemäßen Dehydrier-Katalysator zeichnet sich durch eine verbesserte Ausbeute, beispielsweise eine verbesserte Styrol-Ausbeute, im Vergleich zu bekannten Verfahren bzw. Dehydrier-Katalysatoren, aus.

Bevorzugt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem molaren Dampf/Kohlenwasserstoff-Verhältnis im Bereich von 1 bis 20; bevorzugt von 1 bis 10; insbesondere bevorzugt von 1 bis 9; besonders bevorzugt von 5 bis 8,5; eingesetzt wird. Insbesondere betrifft die Erfindung ein Verfahren zur katalytischen Dehydrierung von Ethylbenzol zu Styrol, wobei ein Gemisch aus Wasserdampf und Ethylbenzol mit einem Dampf/Kohlenwasserstoff-Gewichtsverhältnis im Bereich von 0,17 bis 3,4; bevorzugt von 0,17 bis 1,7; insbesondere bevorzugt von 0,17 bis 1,5; besonders bevorzugt von 0,9 bis 1,45; eingesetzt wird. Insbesondere bevorzugt kann die erfindungsgemäße katalytische Dehydrierung eines Kohlenwasserstoffs mit einem mittleren Dampf/Kohlenwasserstoff-Gewichtsverhältnis im Bereich von 0,9 bis 1,45 (kg/kg) durchgeführt werden.

Typischerweise werden bei dem erfindungsgemäßen Verfahren zur katalytischen Dehydrierung pro Durchgang durch den Reaktor (per-pass) Ausbeuten von 40 bis 80 %, bevorzugt von 50 bis 75 %, besonders bevorzugt von 60 bis 70 % bezogen auf den eingesetzten Kohlenwasserstoff erreicht. Insbesondere werden bei der katalytischen Dehydrierung von Ethylbenzol pro Durchgang durch den Reaktor Styrol-Ausbeuten von 40 bis 80 %, bevorzugt von 50 bis 75 %, besonders bevorzugt von 60 bis 70 % bezogen auf das eingesetzten Ethylbenzol, erreicht. Die angegebenen Ausbeuten beziehen sich auf Mol-%.

Typischerweise wird das Verfahren zur katalytischen Dehydrierung eines Kohlenwasserstoffs bei Temperaturen von 500 bis 650 °C und Drücken von 0,2 bis 2 bar absolut durchgeführt.

Bei den beschriebenen Verfahren kann es sich um die Dehydrierung von alkylaromatischen oder aliphatischen Kohlenwasserstoffen handeln, bevorzugt handelt es sich um die Dehydrierung von alkylaromatischen Kohlenwasserstoffen, besonders bevorzugt um die Dehydrierung von Ethylbenzol zu Styrol. Bei den erfindungsgemäßen Verfahren zur Dehydrierung eines Kohlenwasserstoffs kann es sich beispielsweise um die Dehydrierung von Ethylbenzol zu Styrol, von Isopropylbenzol zu alpha-Methylstyrol, von Buten zu Butadien oder von Isoamylen zu Isopren handeln. Bevorzugt handelt es sich bei dem Kohlenwasserstoff um Ethylbenzol.

Weiterhin bezieht sich die vorliegende Erfindung auf die Verwendung eines oben beschriebenen Dehydrier-Katalysators zur katalytischen Dehydrierung eines Kohlenwasserstoffs, insbesondere eines alkylaromatischen oder aliphatischen Kohlenwasserstoffen, bevorzugt eines alkylaromatischen Kohlenwasserstoffs, besonders bevorzugt von Ethylbenzol. Bevorzugt bezieht sich die Erfindung auf die Verwendung eines oben beschriebenen Dehydrier-Katalysators zur katalytischen Dehydrierung eines Kohlenwasserstoffs bei einem molaren Dampf/ Kohlenwasserstoff-Verhältnis im Bereich von 1 bis 20; bevorzugt im Bereich von 1 bis 10; insbesondere bevorzugt im Bereich von 1 bis 9, besonders bevorzugt im Bereich von 5 bis 8,5.

Im Folgenden wird die Figur erläutert:

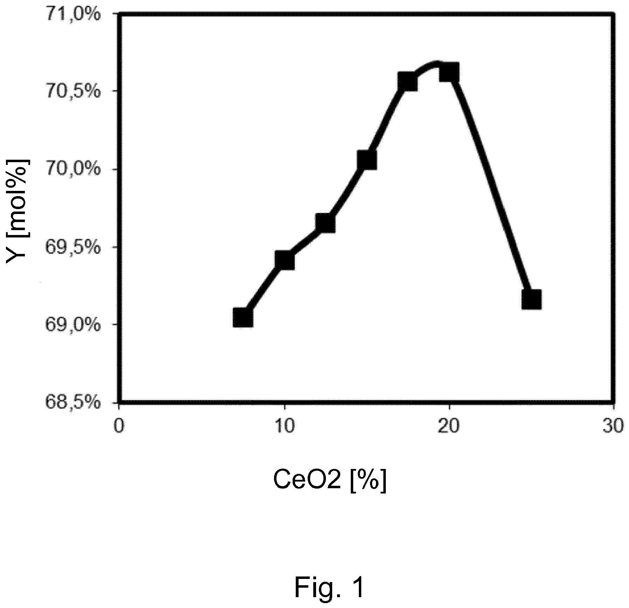

Figur 1 zeigt die Styrol-Ausbeute Y in Mol-% bei der katalytischen Dehydrierung von Ethylbenzol (gemäß Beispielen 1 bis 8) bei einem Wasserdampf/Ethylbenzol-Gewichtsverhältnis von 1,25 kg/kg und einer Temperatur von 620 °C in Abhängigkeit des Gehalts an $CeO_2$ [%] in Gew.-% im eingesetzten Katalysator (bezogen auf den gesamten Katalysator).

Die Styrol-Ausbeute in Mol-% wird jeweils angegeben in Stoffmenge an hergestelltem Styrol bezogen auf Stoffmenge an eingesetztem Ethylbenzol.

Die vorliegende Erfindung soll durch die folgenden Beispiele näher erläutert werden.

Beispiele

Es wurden Katalysatoren hergestellt, die im Wesentlichen die gleiche Zusammensetzung in Bezug auf Kaliumoxid sowie Promotoren aufweisen aber unterschiedliche Mengen Cerdioxid enthalten. Die Variation des Gehaltes an Cerdioxid wurde durch entsprechende Anpassung des Gehaltes an Eisenoxid ausgeglichen. Die Katalysatoren wurden unter gleichen Bedingungen hergestellt.

Die Charakterisierung der Katalysatoren erfolgte mittels Röntgen-Diffraktometrie , wobei der Gehalt an K/Fe-Mischoxidphasen und die $CeO_2$-Kristallitgröße bestimmt wurde (siehe Beispiel 9).

Beispiele 1 bis 3 (Vergleichsbeispiele) - Herstellung der Katalysatoren K1 bis K3

Als Komponenten wurden Eisenoxid (alpha-$Fe_2O_3$, Hämatit), Kaliumcarbonat ($K_2CO_3$), Cercarbonat ($Ce_2CO_3$), Magnesiumoxid (MgO), Molybdänoxid ($MoO_3$), Calciumhydroxid (Ca(OH)$_2$) und Titandioxid ($TiO_2$) eingesetzt.

Die oben genannten pulverförmigen Komponenten wurden zunächst trocken vermischt und dann unter Zugabe von Wasser und Stärkelösung geknetet. Die Katalysatormasse wurde extrudiert, wobei Pellets mit 3 mm Durchmesser erhalten wurden. Die Katalysator-Formkörper (Pellets) wurden 1h bei 120°C und 1 h bei 350°C getrocknet und anschließend 1 h bei 805°C in Luft in einem Muffelofen kalziniert.

Es wurde ein Katalysator K1 mit der folgenden nominalen Oxidzusammensetzung erhalten:

| | |
|---|---|
| 12,0 Gew.-% | $K_2O$, |
| 5,0 Gew.-% | $CeO_2$, |
| 2,1 Gew.-% | MgO, |
| 2,0 Gew.-% | CaO, |
| 2,4 Gew.-% | $MoO_3$, |
| 200 ppm | $TiO_2$ |
| Rest | $Fe_2O_3$ (76,48 Gew.-%) |

Alle Angaben beziehen sich auf Gew.-%, bezogen auf die Gesamtmenge an Katalysator. Alle Angaben beziehen sich - soweit nicht anders angegeben - auf das Oxid des betreffenden Metalls in der höchsten Oxidationsstufe.

Es wurde weitere Katalysatoren (Katalysatoren K2 und K3) wie in Vergleichsbeispiel 1 beschrieben hergestellt, wobei der $CeO_2$-Gehalt variiert wurde (7,5 Gew.% und 10 Gew.-%). Die Änderung des $CeO_2$-Gehaltes wurde durch die Änderung des $Fe_2O_3$-Gehaltes ausgeglichen.

Die Zusammensetzungen der Katalysatoren sind in Tabelle 1 zusammengestellt.

Beispiele 4 bis 8 - Herstellung der Katalysatoren K4 bis K8

Es wurde weitere Katalysatoren (Katalysatoren K4 bis K8) wie in Vergleichsbeispiel 1 beschrieben hergestellt, wobei der $CeO_2$-Gehalt variiert wurde (12,5 Gew.%, 15,0 Gew.%, 17,5 Gew.%, 20,0 Gew.%, 25,0 Gew.%). Die Änderung des $CeO_2$-Gehaltes wurde durch die Änderung des $Fe_2O_3$-Gehaltes ausgeglichen.
Die Zusammensetzungen der Katalysatoren sind in Tabelle 1 zusammengestellt.

Beispiel 9: Charakterisierung der Katalysatoren K1 bis K8

Die kristallographische Phasenzusammensetzungen, z.B. Gehalt an K/Fe-Mischoxidphasen und Cerdioxid-Kristallitgröße, wurden anhand von Röntgen-Diffraktionsmessungen nach folgender Methode bestimmt:
Die Katalysator-Formkörper wurden mit einer Mühle zu einem feinen Pulver gemahlen. Danach wurden die Proben in einen Standard-Probenhalter (Fa. Bruker AXS GmbH) gefüllt und mit einer Glasplatte glatt gestrichen. Die Proben wurden in einem D8 Advance Diffraktometer (Fa. Bruker AXS GmbH) mit einer variablen Blende (V20 - bestrahlte Probenlänge von 20mm) und einem energiedispersiven Punktdetektor (Sol-X, Fa. Bruker AXS GmbH) im Winkelbereich von 10° bis 55° 2θ (2 Theta) mit einer Schrittweite von 0,02° 2θ (2 Theta) vermessen. Die Auswertung der Daten erfolgte mit der Software TOPAS 4.2 (Fa. Bruker AXS GmbH). Die Phasenzusammensetzung der Katalysatoren umfasste typischerweise variable Anteile von verschiedenen kristallographischen Phasen, z.B. Cerianit ($CeO_2$), Hematit ($Fe_2O_3$), Magnetit ($Fe_3O_4$), $K_2CO_3 \cdot 1,5\,H_2O$, $K_4H_2(CO_3)_3 \cdot 1,5\,H_2O$, $KFe_{11}O_{17}$, $K_2Fe_{10}O_{16}$, $K_6Fe_2O_5$, $K_6Fe_2O_6$, $K_9(FeO_4)_2$ (Raumgruppe C2/c) und $K_{17}Fe_5O_{16}$ (Raumgruppe Cm), $K_2Fe_2O_4$, $KFeO_2$ und gegebenenfalls andere oxidische Phasen, welche abhängig sind von den weiteren beinhalteten Metallverbindungen. Bei allen Phasen wurden die Gitterparameter, Kristallitgröße, und Skalierung verfeinert, sowie bei $KFe_{11}O_{17}$ zusätzlich noch die Gaußsche Komponente der Gitterverzerrung (Strain) und eine March-Dollase Vorzugorientierung in Richtung (001). Der Untergrund wurde mit einem Polynom dritter Ordnung angepasst, und der Probenhöhenfehler wurde verfeinert. Intensitätskorrekturen für die Lorentz-Polarisation wurden berücksichtigt. Die Kristallitgröße ist der von der TOPAS Software mit der Bezeichnung "Lvol FWHM" berechnete Wert.
In der folgenden Tabelle 1 sind die nominalen Zusammensetzungen der Katalysatoren, die Anteile an K/Fe-Mischoxidphase und die $CeO_2$-Kristallitgrößen zusammengefasst.

Tabelle 1: Zusammensetzung aller Katalysatoren (Gew.-% als Oxid

| Katalysator | $CeO_2$ | $K_2O$ | MgO | CaO | $MoO_3$ | $TiO_2$ | $Fe_2O_3$ | Mittlere $CeO_2$ Kristallitgröße | K/Fe-Mischoxidphasen |
|---|---|---|---|---|---|---|---|---|---|
| | Gew.-% | | | | | ppm | | nm | Gew.% |
| K1 | 5,0 | 12,0 | 2,1 | 2,0 | 2,4 | 200 | Rest | 25 | 90 |
| K2 | 7,5 | 12,0 | 2,1 | 2,0 | 2,4 | 200 | Rest | 25 | 76 |
| K3 | 10,0 | 12,0 | 2,1 | 2,0 | 2,4 | 200 | Rest | 24 | 74 |
| K4 | 12,5 | 12,0 | 2,1 | 2,0 | 2,4 | 200 | Rest | 23 | 71 |
| K5 | 15,0 | 12,0 | 2,1 | 2,0 | 2,4 | 200 | Rest | 23 | 71 |
| K6 | 17,5 | 12,0 | 2,1 | 2,0 | 2,4 | 200 | Rest | 24 | 68 |
| K7 | 20,0 | 12,0 | 2,1 | 2,0 | 2,4 | 200 | Rest | 23 | 65 |
| K8 | 25,0 | 12,0 | 2,1 | 2,0 | 2,4 | 200 | Rest | 24 | 60 |

Beispiel 10 - Dehydrierung von Ethylbenzol zu Styrol bei einem Dampf/Ethylbenzol Verhältnis von 1,25 kg/kg

Die Katalysatoren K1 bis K3 und K4 bis K8 aus den Beispielen 1 bis 8 wurden in der Dehydrierung von Ethylbenzol zu Styrol in Anwesenheit von Wasserdampf eingesetzt. Das erhaltene Katalysatormaterial wurde zerkleinert und gesiebt, wobei eine Fraktion von 0,5 bis 0,7 mm abgetrennt wurde, welche für die weiteren Versuche verwendet wurde.
Pro Katalysator wurden jeweils zwei isothermen Rohrreaktoren mit je 13,3 ml Katalysator der Fraktion mit einer Korngröße von 0,5 bis 0,7 mm gefüllt. Die Reaktoren wurden jeweils bei 620 °C und 1 atm Ausgangsdruck mit 14,6 g/h Ethylbenzol und 18,3 g/h vollentsalztem (VE) Wasser kontinuierlich beaufschlagt, was einem Wasser/Ethylbenzol (D/KW)-Verhältnis von 1,25 kg/kg oder 7,36 Mol/Mol entspricht. Nach Stabilisierung, etwa nach 40 h, wurden aus dem flüssigen Kondensat Proben genommen und gaschromatographisch analysiert. Umsatz, Selektivität und Styrol-Ausbeute wurden für jeden Reaktor bestimmt. Für jeden Katalysator wurde einen Mittelwert über die beiden parallel geführten Reaktoren ermittelt. Die Ergebnisse werden in Tabelle 2 dargestellt.

Tabelle 2: Katalytische Eigenschaften der Katalysatoren K1 bis K8

| Katalysator | $CeO_2$-Gehalt, | Mittlere $CeO_2$ Kristallitgröße | K/Fe-Mischoxidphasen | Ethylbenzol Umsatz | Styrol Selektivität | Styrol Ausbeute |
|---|---|---|---|---|---|---|
| | Gew.-% | nm | Gew.-% | Mol% | Mol% | Mol% |
| K1 | 5,0 | 25 | 90 | 71,58 | 95,63 | 68,45 |
| K2 | 7,5 | 25 | 76 | 72,22 | 95,61 | 69,05 |
| K3 | 10,0 | 24 | 74 | 72,56 | 95,66 | 69,41 |
| K4 | 12,5 | 23 | 71 | 72,88 | 95,57 | 69,65 |
| K5 | 15,0 | 23 | 71 | 73,36 | 95,51 | 70,06 |
| K6 | 17,5 | 24 | 68 | 74,06 | 95,28 | 70,56 |
| K7 | 20,0 | 23 | 65 | 74,20 | 95,19 | 70,63 |
| K8 | 25,0 | 24 | 60 | 72,93 | 94,84 | 69,16 |

Ethylbenzol-Umsatz, Styrol-Selektivität und -Ausbeute wurden anhand der folgenden Formeln bestimmt:

$$\text{Umsatz (Mol-\%)} = [A*M_f - B*M_p)/(A*M_f)] \times 100$$

$$\text{Selektivität (Mol-\%)} = [D*M_p - C*M_f)/(A*M_f - B*M_p)] \times (M_{EB}/M_{ST}) \times 100$$

$$\text{Ausbeute (Mol-\%)} = \text{Umsatz} \times \text{Selektivität} / 100$$

mit:

A: Ethylbenzol-Konzentration am Reaktor-Eingang (Gew.-%)
B: Ethylbenzol-Konzentration am Reaktor-Ausgang (Gew.-%)
C: Styrol-Konzentration am Reaktor-Eingang (Gew.-%)
D: Styrol-Konzentration am Reaktor-Ausgang (Gew.-%)
$M_f$: Mittlere Molmasse der organischen Edukte
$M_p$: Mittlere Molmasse der organischen Produkte
$M_{EB}$: Molmasse Ethylbenzol
$M_{ST}$: Molmasse Styrol

Die oben genannten Angaben bezüglich Konzentration und Molmassen beziehen sich jeweils auf die organische Phase (ohne Wasser).
Die Daten in Tabelle 2 zeigen, dass mit den Katalysatoren K4 bis K7 mit einem Cerdioxid-Gehalt im Bereich von 12,5 bis 20 Gew.% deutlich höhere Styrol-Ausbeuten erhalten werden als mit den Katalysatoren aus den Vergleichsbeispiele K1 bis K3, welche niedrigere Anteile an Cerdioxid aufweisen. Die Daten in Tabelle 2 zeigen weiterhin, dass ein zu hoher Cerdioxid-Gehalt den Gehalt an K/Fe-Mischoxidphasen vermindert und der Katalysator eine geringere Styrol-Ausbeute liefert (Katalysator 8). Die höchste Ausbeuten werden erhalten bei Verwendung der Katalysatoren aus den Beispielen 5, 6 und 7, welche einen Cerdioxid-Gehalt im Bereich von 15 bis 20 Gew.% und einen Anteil an K/Fe-Mischoxidphasen von mindestens 65 Gew.-% aufweisen.
Weiterhin ist in Tabelle 2 zu erkennen, dass die Partikelgröße der Cerdioxid-Partikel in der Versuchsreihe annähernd gleich bleibt.
In Figur 1 wird die Styrol-Ausbeute Y [%] in Abhängigkeit des Anteils an $CeO_2$ in Gew.-% [%$CeO_2$] im Katalysator dargestellt. Es zeigt sich sehr deutlich, dass im Bereich von 11 bis 24 Gew.-% $CeO_2$ eine optimale Styrol-Ausbeute erhalten werden kann.
Es wurde eine optimaler Gehalt an Cerdioxid im Bereich von 11 bis 24 Gew.-%, bevorzugt von 12,5 bis 22 Gew.-%, insbesondere bevorzugt von 15 bis 20 Gew.-% gefunden.

**Patentansprüche**

1.  Dehydrier-Katalysator enthaltend mindestens eine Eisenverbindung, mindestens eine Kaliumverbindung und 11 bis 24 Gew.-%, bezogen auf den gesamten Katalysator, mindestens einer Cerverbindung, gerechnet als $CeO_2$, wobei die mindestens eine Eisenverbindung und die mindestens eine Kaliumverbindung zumindest teilweise in Form von einer oder mehreren K/Fe-Mischoxidphasen der allgemeinen Formel $K_xFe_yO_z$ vorliegen, wobei x eine Zahl von 1 bis 17 ist; y eine Zahl von 1 bis 22 ist und z eine Zahl von 2 bis 34 ist, wobei der Katalysator 65 bis 89 Gew.-%, bezogen auf den gesamten Katalysator, der einen oder mehreren K/Fe-Mischoxidphasen enthält, wobei der Katalysator Cerdioxid-Kristallite mit einem mittleren Durchmesser von 10 bis 30 nm, enthält, wobei mindestens 50 Gew.-% des Cers, bezogen auf die gesamte Menge des eingesetzten Cers, gerechnet als $CeO_2$, in Form von Cerdioxid-Kristalliten mit einem mittleren Durchmesser von 10 bis 30 nm vorliegen, und wobei der mittlere Durchmesser mittels Röntgen-Diffraktometrie bestimmt wird.

2.  Dehydrier-Katalysator gemäß Anspruch 1, **dadurch gekennzeichnet, dass** der Katalysator 15 bis 20 Gew.-% der mindestens einen Cerverbindung, gerechnet als $CeO_2$, enthält.

3.  Dehydrier-Katalysator gemäß einem der Ansprüche 1 bis 2, **dadurch gekennzeichnet, dass** der Katalysator als weitere Komponente 0,1 bis 20 Gew.-%, mindestens einer Erdalkalimetall-Verbindung, gerechnet als Oxid, enthält.

4.  Dehydrier-Katalysator gemäß einem der Ansprüche 1 bis 3, **dadurch gekennzeichnet, dass** der Katalysator als weitere Komponente 0,0001 bis 10 Gew.-%, mindestens einer Verbindung ausgewählt aus Verbindungen umfassend ein Metall ausgewählt aus der Gruppe bestehend aus Molybdän, Titan, Vanadium und Wolfram, gerechnet als Oxid in der jeweils höchsten Oxidationsstufe, enthält.

5.  Dehydrier-Katalysator gemäß einem der Ansprüche 1 bis 4, **dadurch gekennzeichnet, dass** der Katalysator

    50 bis 80 Gew.-% mindestens einer Eisenverbindung, gerechnet als $Fe_2O_3$;
    1 bis 30 Gew.-% mindestens einer Kaliumverbindung, gerechnet als $K_2O$;
    11 bis 24 Gew.-% mindestens einer Cerverbindung, gerechnet als $CeO_2$;
    0,1 bis 10 Gew.-% mindestens einer Magnesiumverbindung gerechnet als MgO;
    0,1 bis 10 Gew.-% mindestens einer Calciumverbindung, gerechnet als CaO;
    0,0001 bis 10 Gew.-% mindestens einer Verbindung ausgewählt aus Verbindungen umfassend ein Metall ausgewählt aus der Gruppe bestehend aus Molybdän (Mo), Titan (Ti), Vanadium (V) und Wolfram (W), jeweils gerechnet als das Oxid in der höchsten Oxidationsstufe;

    enthält.

6.  Dehydrier-Katalysator gemäß einem der Ansprüche 1 bis 5, **dadurch gekennzeichnet, dass** der Katalysator

    50 bis 80 Gew.-% mindestens einer Eisenverbindung, gerechnet als $Fe_2O_3$;
    1 bis 30 Gew.-% mindestens einer Kaliumverbindung, gerechnet als $K_2O$;
    11 bis 24 Gew.-% mindestens einer Cerverbindung, gerechnet als $CeO_2$;
    0,1 bis 10 Gew.-% mindestens einer Magnesiumverbindung gerechnet als MgO;
    0,1 bis 10 Gew.-% mindestens einer Calciumverbindung, gerechnet als CaO;
    0,1 bis 10 Gew.-% mindestens einer Molybdänverbindung, gerechnet als $MoO_3$;
    1 bis 1.000 ppm mindestens einer Titanverbindung, gerechnet als $TiO_2$;

    enthält.

7.  Dehydrier-Katalysator gemäß einem der Ansprüche 1 bis 6, **dadurch gekennzeichnet, dass** der Katalysator

    50 bis 80 Gew.-% mindestens einer Eisenverbindung, gerechnet als $Fe_2O_3$;
    1 bis 30 Gew.-% mindestens einer Kaliumverbindung, gerechnet als $K_2O$;
    15 bis 20 Gew.-% mindestens einer Cerverbindung, gerechnet als $CeO_2$;
    0,1 bis 10 Gew.-% mindestens einer Magnesiumverbindung gerechnet als MgO;

0,1 bis 10 Gew.-% mindestens einer Calciumverbindung, gerechnet als CaO;
0,1 bis 10 Gew.-% mindestens einer Molybdänverbindung, gerechnet als $MoO_3$;
1 bis 1.000 ppm mindestens einer Titanverbindung, gerechnet als $TiO_2$;

enthält.

**8.** Verfahren zur Herstellung eines Dehydrier-Katalysators gemäß einem der Anspruche 1 bis 7, umfassend die folgenden Schritte

i) Herstellen einer Katalysator-Vormischung durch Vermischen mindestens einer Eisenverbindung, mindestens einer Kaliumverbindung, 11 bis 24 Gew.-%, bezogen auf den fertigen Katalysator, mindestens einer Cerverbindung, gerechnet als $CeO_2$, optional weiterer Metallverbindungen, optional weiterer Komponenten und optional mindestens eines Bindemittels mit einem Lösungsmittel;
ii) Herstellen von Katalysator-Formkörpern aus der in Schritt i) erhaltenen Katalysator-Vormischung;
iii) Trocknen der Katalysator-Formkörper und Kalzinieren der Katalysator-Formkörper,
wobei das Kalzinieren der Katalysator-Formkörper in Schritt iii) bei einer Temperatur im Bereich von 750 bis 850 °C erfolgt.

**9.** Verfahren zur katalytischen Dehydrierung eines Kohlenwasserstoffs, wobei ein Gemisch aus Wasserdampf und mindestens einem Kohlenwasserstoff mit einem Dehydrier-Katalysator gemäß einem der Ansprüche 1 bis 7 in Kontakt gebracht wird.

**10.** Verfahren zur katalytischen Dehydrierung eines Kohlenwasserstoffs gemäß Anspruch 9, **dadurch gekennzeichnet, dass** ein Gemisch aus Wasserdampf und mindestens einem Kohlenwasserstoff mit einem molaren Dampf/Kohlenwasserstoff-Verhältnis im Bereich von 1 bis 10 eingesetzt wird.

**11.** Verfahren zur katalytischen Dehydrierung eines Kohlenwasserstoffs gemäß einem der Ansprüche 9 oder 10, **dadurch gekennzeichnet, dass** es sich bei dem Kohlenwasserstoff um Ethylbenzol handelt.

**12.** Verwendung eines Dehydrier-Katalysators gemäß einem der Ansprüche 1 bis 7 zur katalytischen Dehydrierung eines Kohlenwasserstoffs.

**Claims**

**1.** A dehydrogenation catalyst comprising at least one iron compound, at least one potassium compound and from 11 to 24% by weight, based on the total catalyst, of at least one cerium compound, calculated as $CeO_2$, wherein the at least one iron compound and the at least one potassium compound are at least partly present in the form of one or more K/Fe mixed oxide phases of the general formula $K_xFe_yO_z$, where x is from 1 to 17; y is from 1 to 22 and z is from 2 to 34, where the catalyst comprises from 65 to 89% by weight, based on the total catalyst, of the one or more K/Fe mixed oxide phases, where the catalyst comprises cerium dioxide crystallites having an average diameter of from 10 to 30 nm, where at least 50% by weight of the cerium, based on the total amount of the cerium employed, calculated as $CeO_2$, is present in the form of cerium dioxide crystallites having an average diameter of from 10 to 30 nm, and where the average diameter is determined by means of X-ray diffraction.

**2.** The dehydrogenation catalyst according to claim 1, wherein the catalyst comprises from 15 to 20% by weight of the at least one cerium compound, calculated as $CeO_2$.

**3.** The dehydrogenation catalyst according to any of claims 1 to 2, wherein the catalyst comprises from 0.1 to 20% by weight, of at least one alkaline earth metal compound, calculated as oxide, as further component.

**4.** The dehydrogenation catalyst according to any of claims 1 to 3, wherein the catalyst comprises from 0.0001 to 10% by weight, of at least one compound selected from compounds comprising a metal selected from the group consisting of molybdenum, titanium, vanadium and tungsten, calculated as oxide in the highest oxidation state in each case, as further component.

**5.** The dehydrogenation catalyst according to any of claims 1 to 4, wherein the catalyst comprises

from 50 to 80% by weight of at least one iron compound, calculated as $Fe_2O_3$;
from 1 to 30% by weight of at least one potassium compound, calculated as $K_2O$;
from 11 to 24% by weight of at least one cerium compound, calculated as $CeO_2$;
from 0.1 to 10% by weight of at least one magnesium compound, calculated as $MgO$;
from 0.1 to 10% by weight of at least one calcium compound, calculated as $CaO$;
from 0.0001 to 10% by weight, of at least one compound selected from compounds comprising a metal selected from the group consisting of molybdenum (Mo), titanium (Ti), vanadium (V) and tungsten (W), in each case calculated as the oxide in the highest oxidation state.

6. The dehydrogenation catalyst according to any of claims 1 to 5, wherein the catalyst comprises

from 50 to 80% by weight of at least one iron compound, calculated as $Fe_2O_3$;
from 1 to 30% by weight of at least one potassium compound, calculated as $K_2O$;
from 11 to 24% by weight of at least one cerium compound, calculated as $CeO_2$;
from 0.1 to 10% by weight of at least one magnesium compound, calculated as $MgO$;
from 0.1 to 10% by weight of at least one calcium compound, calculated as $CaO$;
from 0.1 to 10% by weight of at least one molybdenum compound, calculated as $MoO_3$;
from 1 to 1000 ppm of at least one titanium compound, calculated as $TiO_2$.

7. The dehydrogenation catalyst according to any of claims 1 to 6, wherein the catalyst comprises

from 50 to 80% by weight of at least one iron compound, calculated as $Fe_2O_3$;
from 1 to 30% by weight of at least one potassium compound, calculated as $K_2O$;
from 15 to 20% by weight of at least one cerium compound, calculated as $CeO_2$;
from 0.1 to 10% by weight of at least one magnesium compound, calculated as $MgO$;
from 0.1 to 10% by weight of at least one calcium compound, calculated as $CaO$;
from 0.1 to 10% by weight of at least one molybdenum compound, calculated as $MoO_3$;
from 1 to 1000 ppm of at least one titanium compound, calculated as $TiO_2$.

8. A process for producing a dehydrogenation catalyst according to any of claims 1 to 7, which comprises the following steps

i) production of a catalyst premix by mixing of at least one iron compound, at least one potassium compound, from 11 to 24% by weight, based on the finished catalyst, of at least one cerium compound, calculated as $CeO_2$; optionally further metal compounds, optionally further components and optionally at least one binder with a solvent;
ii) production of shaped catalyst bodies from the catalyst premix obtained in step i);
iii) drying of the shaped catalyst bodies and calcination of the shaped catalyst bodies,

wherein the calcination of the shaped catalyst bodies in step iii) is carried out at a temperature in the range from 750 to 850°C.

9. A process for the catalytic dehydrogenation of a hydrocarbon, wherein a mixture of steam and at least one hydrocarbon is brought into contact with a dehydrogenation catalyst according to any of claims 1 to 7.

10. The process for the catalytic dehydrogenation of a hydrocarbon according to claim 9, wherein a mixture of steam and at least one hydrocarbon having a molar steam/hydrocarbon ratio in the range from 1 to 10 is used.

11. The process for the catalytic dehydrogenation of a hydrocarbon according to either claim 9 or 10, wherein the hydrocarbon is ethylbenzene.

12. The use of a dehydrogenation catalyst according to any of claims 1 to 7 for the catalytic dehydrogenation of a hydrocarbon.

**Revendications**

1. Catalyseur de déshydrogénation contenant au moins un composé de fer, au moins un composé de potassium et 11 à 24 % en poids, par rapport à l'ensemble du catalyseur, d'au moins un composé de cérium, calculé en tant que CeO$_2$, ledit au moins un composé de fer et ledit au moins un composé de potassium se présentant au moins partiellement sous la forme d'une ou de plusieurs phases d'oxyde mixte K/Fe de la formule générale K$_x$Fe$_y$O$_z$, x étant un nombre de 1 à 17, y étant un nombre de 1 à 22, et z étant un nombre de 2 à 34, le catalyseur contenant 65 à 89 % en poids, par rapport à l'ensemble du catalyseur, de ladite ou desdites phases d'oxyde mixte K/Fe, le catalyseur contenant des cristallites de dioxyde de cérium ayant un diamètre moyen de 10 à 30 nm, au moins 50 % en poids du cérium, par rapport à la quantité totale du cérium utilisé, calculé en tant que CeO$_2$, se présentant sous la forme de cristallites de dioxyde de cérium ayant un diamètre moyen de 10 à 30 nm, et le diamètre moyen étant déterminé par diffractométrie de rayons X.

2. Catalyseur de déshydrogénation selon la revendication 1, **caractérisé en ce que** le catalyseur contient 15 à 20 % en poids dudit au moins un composé de cérium, calculé en tant que CeO$_2$.

3. Catalyseur de déshydrogénation selon l'une quelconque des revendications 1 2, **caractérisé en ce que** le catalyseur contient en tant que composant supplémentaire 0,1 à 20 % en poids d'au moins un composé de métal alcalino-terreux, calculé en tant qu'oxyde.

4. Catalyseur de déshydrogénation selon l'une quelconque des revendications 1 3, **caractérisé en ce que** le catalyseur contient en tant que composant supplémentaire 0,0001 à 10 % en poids d'au moins un composé choisi parmi les composés comprenant un métal choisi dans le groupe constitué par le molybdène, le titane, le vanadium et le tungstène, calculé en tant qu'oxyde à chaque fois au niveau d'oxydation le plus élevé.

5. Catalyseur de déshydrogénation selon l'une quelconque des revendications 1 4, **caractérisé en ce que** le catalyseur contient :

50 à 80 % en poids d'au moins un composé de fer, calculé en tant que Fe$_2$O$_3$ ;
1 à 30 % en poids d'au moins un composé de potassium, calculé en tant que K$_2$O ;
11 à 24 % en poids d'au moins un composé de cérium, calculé en tant que CeO$_2$ ;
0,1 à 10 % en poids d'au moins un composé de magnésium, calculé en tant que MgO ;
0,1 à 10 % en poids d'au moins un composé de calcium, calculé en tant que CaO ;
0,0001 à 10 % en poids d'au moins un composé choisi parmi les composés comprenant un métal choisi dans le groupe constitué par le molybdène (Mo), le titane (Ti), le vanadium (V) et le tungstène (W), à chaque fois calculé sous la forme de l'oxyde au niveau d'oxydation le plus élevé.

6. Catalyseur de déshydrogénation selon l'une quelconque des revendications 1 à 5, **caractérisé en ce que** le catalyseur contient :

50 à 80 % en poids d'au moins un composé de fer, calculé en tant que Fe$_2$O$_3$ ;
1 à 30 % en poids d'au moins un composé de potassium, calculé en tant que K$_2$O ;
11 à 24 % en poids d'au moins un composé de cérium, calculé en tant que CeO$_2$ ;
0,1 à 10 % en poids d'au moins un composé de magnésium, calculé en tant que MgO ;
0,1 à 10 % en poids d'au moins un composé de calcium, calculé en tant que CaO ;
0,1 à 10 % en poids d'au moins un composé de molybdène, calculé en tant que MoO$_3$ ;
1 à 1 000 ppm d'au moins un composé de titane, calculé en tant que TiO$_2$.

7. Catalyseur de déshydrogénation selon l'une quelconque des revendications 1 à 6, **caractérisé en ce que** le catalyseur contient :

50 à 80 % en poids d'au moins un composé de fer, calculé en tant que Fe$_2$O$_3$ ;
1 à 30 % en poids d'au moins un composé de potassium, calculé en tant que K$_2$O ;
15 à 20 % en poids d'au moins un composé de cérium, calculé en tant que CeO$_2$ ;
0,1 à 10 % en poids d'au moins un composé de magnésium, calculé en tant que MgO ;
0,1 à 10 % en poids d'au moins un composé de calcium, calculé en tant que CaO ;
0,1 à 10 % en poids d'au moins un composé de molybdène, calculé en tant que MoO$_3$ ;
1 à 1 000 ppm d'au moins un composé de titane, calculé en tant que TiO$_2$.

8. Procédé de fabrication d'un catalyseur de déshydrogénation selon l'une quelconque des revendications 1 à 7, comprenant les étapes suivantes :

i) la fabrication d'un pré-mélange de catalyseur par mélange d'au moins un composé de fer, d'au moins un composé de potassium, de 11 à 24 % en poids, par rapport au catalyseur fini, d'au moins un composé de cérium, calculé en tant que $CeO_2$, éventuellement d'autres composés métalliques, éventuellement d'autres composants et éventuellement d'au moins un liant avec un solvant ;

ii) la fabrication de corps moulés de catalyseur à partir du pré-mélange de catalyseur obtenu à l'étape i) ;

iii) le séchage des corps moulés de catalyseur et la calcination des corps moulés de catalyseur,

la calcination des corps moulés de catalyseur à l'étape iii) ayant lieu à une température dans la plage allant de 750 à 850 °C.

9. Procédé de déshydrogénation catalytique d'un hydrocarbure, dans lequel un mélange de vapeur d'eau et d'au moins un hydrocarbure est mis en contact avec un catalyseur de déshydrogénation selon l'une quelconque des revendications 1 à 7.

10. Procédé de déshydrogénation catalytique d'un hydrocarbure selon la revendication 9, **caractérisé en ce qu'**un mélange de vapeur d'eau et d'au moins un hydrocarbure ayant un rapport molaire vapeur/hydrocarbure dans la plage allant de 1 à 10 est utilisé.

11. Procédé de déshydrogénation catalytique d'un hydrocarbure selon l'une quelconque des revendications 9 ou 10, **caractérisé en ce que** l'hydrocarbure consiste en l'éthylbenzène.

12. Utilisation d'un catalyseur de déshydrogénation selon l'une quelconque des revendications 1 à 7 pour la déshydrogénation catalytique d'un hydrocarbure.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0181999 A
- US 4460706 A
- US 4758543 A
- EP 0894528 A
- US 6551958 B
- EP 2106852 A1
- WO 9949966 A

**In der Beschreibung aufgeführte Nicht-Patentliteratur**

- **HIRANO.** *Appl. Catal.,* 1986, vol. 28, 119-130